# EUROPEAN PATENT APPLICATION

(11) **EP 4 060 625 A1**
(43) Date of publication of application: **21.09.2022**
(21) Application number: 21193963.2
(22) Date of filing: 31.08.2021
(51) Int. Cl.: G06V 10/82, G06V 40/18, G06V 10/70, A61B 5/00, G06K 9/62

(54) **DETECTING METHOD AND DETECTING APPARATUS RELATED TO IMAGE**

(30) Priority: 19.03.2021 TW 110109981
(71) Applicant: Acer Medical Inc., New Taipei City 22181 (TW)
(72) Inventor: CHEN, Ming-Ke, 221 New Taipei City (TW); TSAI, Chin-Han, 221 New Taipei City (TW)
(74) Representative: 2K Patentanwälte Blasberg Kewitz & Reichel

(57) **Abstract**

A detecting method and a detecting apparatus (100) related to an image are provided. The detecting method includes the following steps. Feature extraction is performed on a to-be-evaluated image to obtain a feature map. The feature map is detected by a classifier to obtain a classification result. The classifier is trained by a deep neural network (DNN) structure. The DNN structure includes a modified connected layer (CL1, CL2). The number of neurons is successively reduced from an input layer (L2-1) to an output layer (L2-N) in the modified connected layer (CL1, CL2). Accordingly, accuracy and efficiency can be improved.

## Description

### BACKGROUND

### Technical Field

The invention relates to an image recognition technology, and more particularly, to a detecting method and a detecting apparatus related to an image.

### Description of Related Art

Medical images are images obtained from photography on specific parts of a living body. These images may be employed to assess the risk of suffering a disease or the severity of a disease. For example, through fundus photography examinations, diseases such as retinopathy, glaucoma, maculopathy, or other diseases can be detected early. Generally speaking, most doctors judge lesions in the medical images manually. Although computer-aided evaluation on medical images is now available, breakthroughs may still be required in indicators such as efficiency, complexity, and accuracy.

### SUMMARY

In light of the above, the embodiments of the invention provide a detecting method and a detecting apparatus related to an image, which is aided by a neural network (NN) structure and improves the same to increase efficiency and accuracy in detection.

In an embodiment of the invention, a detecting method is applicable to a medical image. The detecting method includes (but is not limited to) the following steps. Feature extraction is performed on a to-be-evaluated image to obtain a feature map. The feature map is detected by a classifier to obtain a classification result. The classifier is trained by a deep neural network (DNN) structure. The DNN structure includes a modified connected layer. A number of neurons is successively reduced from an input layer to an output layer in the modified connected layer.

In an embodiment of the invention, a detecting apparatus is applicable to a medical image. The detecting apparatus includes (but is not limited to) a storage device and a processor. The storage device stores a programming code. The processor is coupled to the storage device. The processor is loaded with and executes the programming code to be configured to perform feature extraction on a to-be-evaluated image to obtain at least one feature map, and detect the at least one feature map by a classifier to obtain a classification result. The classifier is trained by a deep neural network structure. The deep neural network structure comprises a modified connected layer. A number of neurons is successively reduced from an input layer to an output layer in the modified connected layer.

Based on the foregoing, according to the embodiments of the invention, in the detecting method and detecting apparatus related to an image, inference may be made on the classes corresponding to the images by utilizing the deep neural network structure with successively reduced channels. Accordingly, noise can be filtered, efficiency can be increased, and inference accuracy can be improved.

To make the aforementioned features and advantages of this invention more comprehensible, several embodiments accompanied with drawings are described in detail as follows.

### BRIEF DESCRIPTION OF THE DRAWINGS

FIG. 1 is a block diagram of elements of a detecting apparatus according to an embodiment of the invention.
FIG. 2 is a flowchart of a detecting method according to an embodiment of the invention.
FIG. 3 is a flowchart of an Inception version 4 model according to an embodiment of the invention.
FIG. 4A is a schematic diagram of a modified connected layer according to an embodiment of the invention.
FIG. 4B is a schematic diagram of a modified connected layer according to another embodiment of the invention.
FIG. 5 is a schematic diagram of the ensemble according to an embodiment of the invention.

### DESCRIPTION OF THE EMBODIMENTS

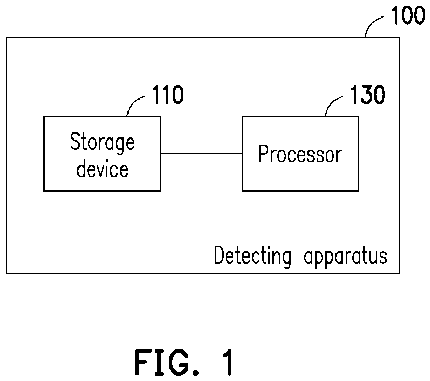

FIG. 1 is a block diagram of elements of a detecting apparatus 100 according to an embodiment of the invention. With reference to FIG. 1, the detecting apparatus 100 includes (but is not limited to) a storage device 110 and a processor 130. The detecting apparatus 100 may be a desktop computer, notebook computer, smart phone, tablet computer, server, medical testing instrument, or other computing devices.

The storage device 110 may be any form of fixed or mobile random access memory (RAM), read only memory (ROM), flash memory, hard disk drive (HDD), solid-state drive (SSD), or similar elements. In an embodiment, the storage device 110 is configured to record programming codes, software modules, configurations, data (e.g., images, feature maps, neural network parameters, neural network (NN) modules, etc.), or files, of which the embodiment will be described in detail later.

The processor 130 is coupled to the storage device 110. The processor 130 may be a central processing unit (CPU), graphics processing unit (GPU), or other programmable general-purpose or special-purpose microprocessor, digital signal processor (DSP), programmable controller, field programmable gate array (FPGA), application-specific integrated circuit (ASIC), neural network accelerator, or other similar elements or a combination of the above elements. In an embodiment, the processor 130 is configured to execute all or part of operations of the detecting apparatus 100, and may be loaded with and execute various programming codes, software modules, files, and data stored in the storage device 110.

Hereinafter, the method according to the embodiment of the invention will be described accompanied with various devices, elements, and modules in the detecting apparatus 100. Each process flow of the method may be adjusted depending on the implementation circumstances, and is not limited thereto.

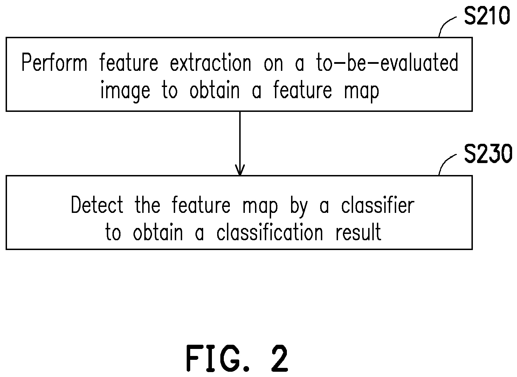

FIG. 2 is a flowchart of a detecting method according to an embodiment of the invention. With reference to FIG. 2, the processor 130 may perform feature extraction on a to-be-evaluated image to obtain one or more feature maps (step S210). Specifically, the to-be-evaluated image may be a medical image, a monitor screen image, or an image in other forms. The to-be-evaluated image is obtained from photography on one or more subjects. Depending on different design requirements, the form, body, and/or content of the to-be-evaluated image may be different. For example, the to-be-evaluated image is a medical image (i.e., fundus image) for fundus photography. For another example, the to-be-evaluated image is a thoracic ultrasound image. For still another example, the to-be-evaluated image is a monitor screen image of entrances and exits.

On the other hand, feature extraction may be applied in machine learning, pattern recognition, image processing, or other technical fields. Feature extraction is to construct an informative and non-redundant derivatives (or referred to as features) for data samples initially measured/collected/extracted. Feature extraction assists subsequent learning and rule induction processes, and provides better interpretation on the initial data samples. In other words, feature extraction may simplify the input data into a feature set (which may be regarded as important or useful information), and the feature set may be directly adopted to perform subsequent tasks (e.g., model training, component analysis, object detection). For example, through feature extraction on images, features such as edges, corners, scale-invariant feature transform (SIFT), curvature, and shape may be obtained.

In an embodiment, the processor 130 may perform feature extraction on the to-be-evaluated image through an artificial neural network (ANN). For example, the processor 130 may perform feature extraction adopting Inception model in any version (e.g., Inception version 4 (v4)), residual neural network (ResNet), GoogLeNet, Visual Geometry Group (VGG), variants thereof, or other image-related networks.

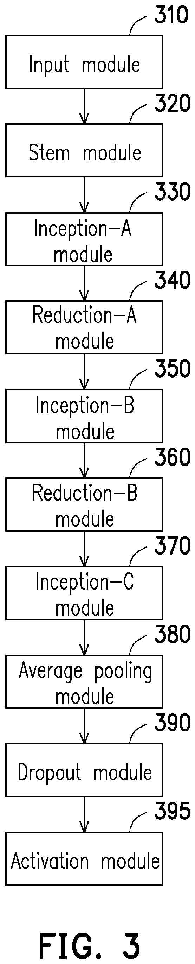

With an Inception v4 model taken as an example, FIG. 3 is a flowchart of an Inception version 4 model according to an embodiment of the invention. With reference to FIG. 3, the following modules may be executed by the processor 130. An input module 310 inputs a to-be-evaluated image. A stem module 320 uses the parallel structure and the asymmetric convolution kernel structure as used in Inception version 3 (v3). An Inception-A module 330, an Inception-B module 350, and an Inception-C module 370 also extract features respectively through different parallel and asymmetric convolution kernel structures. A reduction-A module 340 and a reduction-B module 360 may reduce the size of a feature map. An average pooling module 380 averages adjacent feature points in several regions in the feature map. A dropout module 390 ignores part of neurons in a learning stage. An activation module 395 uses an activation function (e.g., Softmax, Sigmoid, or Rectified Linear Unit (ReLU)) to introduce a non-linear relationship.

In other embodiments, the processor 130 may perform feature extraction on the to-be-evaluated image by using, for example, histogram of oriented gradient (HOG), Harr, speeded up robust features (SURF), or other feature extraction algorithms.

In an embodiment, the feature map is the output after convolution summation performed through a convolution kernel/filter. In another embodiment, the feature map is obtained by recording the feature set/vector obtained from the feature extraction algorithm in the form of a two-dimensional picture.

The processor 130 may detect one or more feature maps through a classifier to obtain a classification result (step S230). Specifically, the classifier is trained by a deep neural network (DNN) structure. The deep neural network adapts an artificial neural network as the main structure. The deep neural network structure includes a modified connected layer. The modified connected layer includes an input layer, hidden layers, and an output layer. The deep neural network is formed by a multi-layer neuron structure. Each layer of neurons is configured with an input (e.g., the output of the previous layer of neurons) and an output. By the neurons in any one of the hidden layers, the inner product of the input vector and the weighted vector is obtained and a scalar result is output through a nonlinear transfer function. In the learning stage of the classifier, the weighted vector is trained and determined, and in the inference stage of the classifier, the classification result is obtained by using the determined weighted vector. The classification result is related to one or more classes or labels to which the to-be-evaluated image pertains.

In the embodiment of the invention, the number of neurons is successively reduced from the input layer to the output layer (i.e., channels are successively reduced) in the modified connected layer. Assuming that the modified connected layer includes an M number of hidden layers, then the number of neurons in the j+1^{th} hidden layer is less than the number of neurons in the j^{th} layer, where M is a positive integer greater than 1, and j is a positive integer between 1 and M. In other words, from the input layer to the output layer, the number of neurons in the next layer is less than the number of neurons in the previous layer.

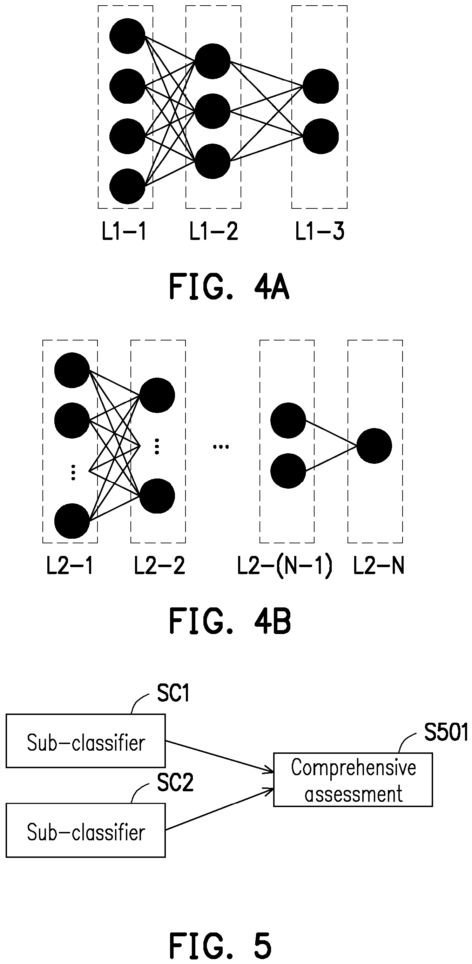

For example, FIG. 4A is a schematic diagram of a modified connected layer according to an embodiment of the invention. With reference to FIG. 4A, a modified connected layer CL1 includes three layers. A first layer L1-1 includes four neurons, a second layer L1-2 includes three neurons, and a third layer L1-3 includes two neurons.

In an embodiment, the modified connected layer includes an O number of hidden layers, and the number of neurons in the k^{th} hidden layer is an integer multiple of the number of neurons in the k+1^{th} hidden layer, where O is a positive integer greater than 1, and k is a positive integer between 1 and O. The integer multiple may be 2 times, 4 times, 8 times, or other multiples.

For example, FIG. 4B is a schematic diagram of a modified connected layer according to another embodiment of the invention. With reference to FIG. 4B, a modified connected layer CL2 includes an N number of layers, and the number of neurons in the i^{th} layer is an integer multiple of the number of neurons in the i+1^{th} layer, where N is a positive integer greater than 1, and i is a positive integer between 1 and N. For example, an input layer L2-1 as the first layer includes 1024 neurons, and a hidden layer L2-2 as the second layer includes 512 neurons. By analogy, a hidden layer L2-(N-1) as the N-1^{th} layer includes 2 neurons, and an output layer L2-N as the N^{th} layer includes one neuron.

In another embodiment, the numbers of neurons are sequentially reduced from the input layer to the output layer in the modified connected layer. For example, the first layer includes 8 neurons, the second layer includes 6 neurons, and so on.

In still another embodiment, the number of neurons reduced is not regular. For example, the first layer includes 10 neurons, the second layer includes 7 neurons, and the third layer includes 2 neurons.

Noted that, the number of layers in the modified connected layer and the number of neurons in each layer may still be changed depending on actual requirements.

In an embodiment, the processor 130 activates each of the neurons in the modified connected layer for the learning stage and the inference stage of the classifier. Specifically, as shown in FIG. 3, the existing Inception v4 randomly deactivates neurons through the dropout module 390 in the learning stage to reduce the possibility of overfitting. In the embodiment of the invention, by replacing the dropout module 390 (i.e., disabling the dropout; possibly also cancelling the activation module 395) with the deep neural network structure (i.e., modified connected layer) with successively reduced channels, noise filtering may be achieved and dimensionality may be reduced. In other words, the learning stage and the inference stage of the classifier are configured that each of the neurons are activated in the modified connected layer, without randomly deactivating neurons.

In an embodiment, the number of neurons in the output layer in the modified connected layer is one. The classification result includes a value, and the value corresponds to one of several classes (or referred to as labels). The classes are results that may be output by the classifier. With age-related maculopathy taken as an example, the value falls within a range, the range is divided into four sections, and each section corresponds to a certain level of severity.

In another embodiment, the output layer of the modified connected layer includes a plurality of neurons, and each neuron in the output layer represents the probability or possibility of being detected as the corresponding class.

In an embodiment, the classifier includes several sub-classifiers, and the sub-classifiers are trained by using the same training data. The sub-classifiers have the same structure and each include the modified connected layer. Even if the same training data is used, two sub-classifiers with the same structure may still obtain different weight parameters. The processor 130 may determine the classification result according to the outputs of the sub-classifiers. For example, the classification results of the sub-classifiers are each a numerical value. Accordingly, the processor 130 may take the average value, maximum value, median value, or other representative values of the classification results of the sub-classifiers as the classification result of the classifier.

FIG. 5 is a schematic diagram of the ensemble according to an embodiment of the invention. With reference to FIG. 5, two sub-classifiers SC1 and SC2 each detect the same feature map, and comprehensive assessment is performed on two output classification results (step S501). The comprehensive assessment is, for example, an arithmetic average, weighted average, or other mathematical expressions.

In an embodiment, the sub-classifiers used by the processor 130 is selected in advance. The selected sub-classifiers have preferred or the most preferred results (e.g., close to or same as the actual results) in both the test data set and the training data set. In addition, the unselected sub-classifiers have less preferred or the least preferred results (e.g., away from the actual results) in the test data set and the training data set.

In summary of the foregoing, in the embodiments of the invention, in the detecting method and the detecting apparatus related to an image, the modified connected layer with successively reduced channels is used to filter impurities and increase efficiency. In addition, the classification results of sub-classifiers may be comprehensively assessed, and the final classification result may accordingly be obtained. Accordingly, sensitivity and specificity can be increased. In the application to medical image testing, it may help doctors or the people to quickly obtain the test results, saving a great amount of test time.

### REFERENCE SIGN LIST

- 100:: detecting apparatus
- 110:: storage device
- 130:: processor
- S210 to S230, S501:: step
- 310:: input module
- 320:: stem module
- 330:: inception-A module
- 340:: reduction-A module
- 350:: inception -B module
- 360:: reduction-B module
- 370:: inception-C module
- 380:: average pooling module
- 390:: dropout module
- 395:: activation module
- CL1, CL2:: modified connected layer
- L1-1:: first layer
- LI-2:: second layer
- L1-3:: third layer
- L2-1:: input layer
- L2-2, L2-(N-1):: hidden layer
- L2-N:: output layer
- SC1, SC2:: sub-classifier

## Claims

1. A detecting method, applicable to a medical image, comprising:
performing a feature extraction on a to-be-evaluated image to obtain at least one feature map; and
detecting the at least one feature map by a classifier to obtain a classification result, wherein the classifier is trained by a deep neural network structure, the deep neural network structure comprises a modified connected layer (CL1, CL2), and a number of neurons is successively reduced from an input layer (L2-1) to an output layer (L2-N) in the modified connected layer (CL1, CL2).

2. The detecting method as described in claim 1, wherein the step of detecting the at least one feature map by the classifier comprises:
activating each of neurons in the modified connected layer (CL1, CL2) for a learning stage and an inference stage of the classifier.

3. The detecting method as described in any of the claim 1 to 2, wherein the modified connected layer (CL1, CL2) comprises an O number of hidden layers (L2-2, L2-(N-1)), and a number of neurons in a k^{th} hidden layer (L2-2, L2-(N-1)) is an integer multiple of a number of neurons in a k+1^{th} hidden layer (L2-2, L2-(N-1)), where O is a positive integer greater than 1, and k is a positive integer between 1 and O.

4. The detecting method as described in any of the claims 1 to 3, wherein the step of detecting the at least one feature map by the classifier comprises:
configuring a number of neurons in the output layer (L2-N) in the modified connected layer (CL1, CL2) as one, wherein the classification result comprises a value, and the value corresponds to one of a plurality of classes.

5. The detecting method as described in any of the claims 1 to 4, wherein the medical image is directed to fundus photography, and the step of performing the feature extraction on the to-be-evaluated image comprises:
performing the feature extraction through an Inception model.

6. The detecting method as described in any of the claims 1 to 5, wherein the classifier comprises a plurality of sub-classifiers (SC1, SC2), the sub-classifiers (SC1, SC2) are trained by using same training data, and the step of detecting the at least one feature map by the classifier comprises:
determining the classification result according to an output of the sub-classifiers (SC1, SC2).

7. A detecting apparatus (100), applicable to a medical image, comprising:
a storage device (110), storing a programming code; and
a processor (130), coupled to the storage device (110), the processor (130) being loaded with and executing the programming code to be configured to:
perform a feature extraction on a to-be-evaluated image to obtain at least one feature map; and
detect the at least one feature map by a classifier to obtain a classification result, wherein the classifier is trained by a deep neural network structure, the deep neural network structure comprises a modified connected layer (CL1, CL2), and a number of neurons is successively reduced from an input layer (L2-1) to an output layer (L2-N) in the modified connected layer (CL1, CL2).

8. The detecting apparatus (100) as described in claim 7, wherein a learning stage and an inference stage of the classifier are configured that each of neurons in the modified connected layer (CL1, CL2) are activated.

9. The detecting apparatus (100) as described in claim 7 or 8, wherein the modified connected layer (CL1, CL2) comprises an O number of hidden layers (L2-2, L2-(N-1)), and a number of neurons in a k^{th} hidden layer (L2-2, L2-(N-1)) is an integer multiple of a number of neurons in a k+1^{th} hidden layer (L2-2, L2-(N-1)), where O is a positive integer greater than 1, and k is a positive integer between 1 and O.

10. The detecting apparatus (100) as described in any of the claims 7 to 9, wherein a number of neurons in the output layer (L2-N) in the modified connected layer (CL1, CL2) is one, the classification result comprises a value, and the value corresponds to one of a plurality of classes.

11. The detecting apparatus (100) as described in any of the claims 7 to 10, wherein the medical image is directed to fundus photography, and the processor (130) is further configured to:
perform the feature extraction through an Inception model.

12. The detecting apparatus (100) as described in any of the claims 7 to 11, wherein the classifier comprises a plurality of sub-classifiers (SC1, SC2), the sub-classifiers (SC1, SC2) are trained by using same training data, and the processor is further configured to:
determine the classification result according to an output of the sub-classifiers (SC1, SC2).
